# EUROPEAN PATENT SPECIFICATION

(11) **EP 1 646 397 B1**
(45) Date of publication and mention of the grant of the patent: **22.08.2012**
(21) Application number: 04777620.8
(22) Date of filing: 06.07.2004
(51) Int. Cl.: A61K 38/21, C07K 14/565

(54) **IMPROVED RECOMBINANT HUMAN INTERFERON-BETA-1b POLYPEPTIDES**
VERBESSERTE REKOMBINANTE HUMANE INTERFERON-BETA-1B POLYPEPTIDE
POLYPEPTIDES DE L'INTERFERON-BETA-1B HUMAINS RECOMBINANTS AMELIORES

(30) Priority: 11.07.2003 US 486657 P
(43) Date of publication of application: 19.04.2006
(73) Proprietor: Bayer Pharma Aktiengesellschaft, 13353 Berlin (DE)
(72) Inventor: NESTAAS, Eirik, Orinda, CA 94563 (US); PUNGOR, Erno, Millbrae, CA 94030 (US)
(74) Representative: Pohlman, Sandra M.
(86) International application number: PCT/US2004/021618
(87) International publication number: WO 2005/016371

(56) References cited:
- WO-A-03/006053
- US-A- 4 572 798
- ETO TOSHIHARU ET AL: "Enhanced inhibition of hepatitis B virus production by asialoglycoprotein receptor-directed interferon" NATURE MEDICINE, vol. 5, no. 5, May 1999 (1999-05), pages 577-581, XP002308496 ISSN: 1078-8956
- KUSHNARYOV V M ET AL: "ULTRASTRUCTURAL DISTRIBUTION OF INTERFERON RECEPTOR SITES ON MOUSE L FIBROBLASTS GROWN IN SUSPENSION GANGLIOSIDE BLOCKADE OF LIGAND BINDING" INFECTION AND IMMUNITY, vol. 40, no. 1, 1983, pages 320-329, XP002308497 ISSN: 0019-9567
- FUSE A ET AL: "EFFECT OF GLYCO LIPIDS AND GLYCOPHORIN ON THE ACTIVITY OF HUMAN INTERFERON BETA AND INTERFERON GAMMA" ANTIVIRAL RESEARCH, vol. 2, no. 3, 1982, pages 161-166, XP002308498 ISSN: 0166-3542 cited in the application
- PARK Y S ET AL: "EFFECT OF CHEMICALLY MODIFIED GM1 AND NEOGLYCOLIPID ANALOGS OF GM1 ON LIPOSOME CIRCULATION TIME: EVIDENCE SUPPORTING THE DYSOPSONIN HYPOTHESIS" BIOCHIMICA ET BIOPHYSICA ACTA, AMSTERDAM, NL, vol. 1166, no. 1, 1993, pages 105-114, XP000672944 ISSN: 0006-3002
- VARTANIAN TIMOTHY: "An examination of the results of the EVIDENCE, INCOMIN, and Phase III studies of interferon beta products in the treatment of multiple sclerosis." CLINICAL THERAPEUTICS, vol. 25, no. 1, January 2003 (2003-01), pages 105-118, XP002308499 ISSN: 0149-2918 cited in the application

### SEQUENCE LISTING

<110> Nestaas, Eirik Pungor, Erno
<120> Improved Recombinant Human Interferon-beta-lb Polypeptides
<130> 53117AWOM1
<160> 2
<170> PatentIn version 3.2
<210> 1
   <211> 166
   <212> PRT
   <213> Homo sapiens
<400> 1
   Met Ser Tyr Asn Leu Leu Gly Phe Leu Gln Arg Ser Ser Asn Phe Gln
   1               5                   10                  15
   Cys Gln Lys Leu Leu Trp Gln Leu Asn Gly Arg Leu Glu Tyr Cys Leu
               20                  25                  30
   Lys Asp Arg Met Asn Phe Asp Ile Pro Glu Glu Ile Lys Gln Leu Gln
           35                  40                  45
   Gln Phe Gln Lys Glu Asp Ala Ala Leu Thr Ile Tyr Glu Met Leu Gln
       50                  55                  60
   Asn Ile Phe Ala Ile Phe Arg Gln Asp Ser Ser Ser Thr Gly Trp Asn
   65                  70                  75                  80
   Glu Thr Ile Val Glu Asn Leu Leu Ala Asn Val Tyr His Gln Ile Asn
                   85                  90                  95
   His Leu Lys Thr Val Leu Glu Glu Lys Leu Glu Lys Glu Asp Phe Thr
               100                 105                 110
   Arg Gly Lys Leu Met Ser Ser Leu His Leu Lys Arg Tyr Tyr Gly Arg
           115                 120                 125
   Ile Leu His Tyr Leu Lys Ala Lys Glu Tyr Ser His Cys Ala Trp Thr
       130                 135                 140
   Ile Val Arg Val Glu Ile Leu Arg Asn Phe Tyr Phe Ile Asn Arg Leu
   145                 150                 155                 160
   Thr Gly Tyr Leu Arg Asn
                   165
<210> 2
   <211> 166
   <212> PRT
   <213> Homo sapiens
<400> 2
   Met Ser Tyr Asn Leu Leu Gly Phe Leu Gln Arg Ser Ser Asn Phe Gln
   1               5                   10                  15
   Ser Gln Lys Leu Leu Trp Gln Leu Asn Gly Arg Leu Glu Tyr Cys Leu
               20                  25                  30
   Lys Asp Arg Met Asn Phe Asp Ile Pro Glu Glu Ile Lys Gln Leu Gln
           35                  40                  45
   Gln Phe Gln Lys Glu Asp Ala Ala Leu Thr Ile Tyr Glu Met Leu Gln
       50                  55                  60
   Asn Ile Phe Ala Ile Phe Arg Gln Asp Ser Ser Ser Thr Gly Trp Asn
   65                  70                  75                  80
   Glu Thr Ile Val Glu Asn Leu Leu Ala Asn Val Tyr His Gln Ile Asn
                   85                  90                  95
   His Leu Lys Thr Val Leu Glu Glu Lys Leu Glu Lys Glu Asp Phe Thr
               100                 105                 110
   Arg Gly Lys Leu Met Ser Ser Leu His Leu Lys Arg Tyr Tyr Gly Arg
           115                 120                 125
   Ile Leu His Tyr Leu Lys Ala Lys Glu Tyr Ser His Cys Ala Trp Thr
       130                 135                 140
   Ile Val Arg Val Glu Ile Leu Arg Asn Phe Tyr Phe Ile Asn Arg Leu
   145                 150                 155                 160
   Thr Gly Tyr Leu Arg Asn
                   165

## Description

This application claims the benefit of U.S. Provisional Application Serial No. 60/486,657, filed on July 11, 2003.

### FIELD OF THE INVENTION

The present invention relates to recombinant human interferon-β-1b ("IFN-β-1 b") polypeptides, or fragments, analogs, derivatives, or variants thereof, having an improved specific activity. The present invention also relates to pharmaceutical compositions comprising such IFN-β-1 b polypeptides, or fragments, analogs, derivatives, or variants thereof, useful for treating multiple sclerosis. The present invention further relates to methods of producing such IFN-β-1b compositions.

### BACKGROUND OF THE INVENTION

Recombinant human interferon-β ("IFN-β") is used clinically to treat certain medical conditions, in particular, the relapsing remitting form of multiple sclerosis ("MS"). Human IFN-β is a single gene lacking introns, and is a member of a large family of secreted proteins known as interferons ("IFNs"), which are cytokines with antiviral, anti-protozoal, immunomodulatory, and cell growth regulatory activities. See, for example, Pestka, S. (1986) Meth. Enzymol. 119:3-14; and Sen, G.C. and Lengyel, P. (1992) J. Biol. Chem. 267:5017-5020.

The polynucleotide and amino acid sequences of human IFN-β are known, as shown in Taniguchi, T. et al. (1980a) Gene 10:11-15 and U.S. Patent No. 5,326,859. Recombinant DNA technology makes it possible to produce an IFN-β that is free from viruses, *e.g.*, HIV-1, and other contaminants from human sources. By means of such technology, human IFN-β can be produced by culturing a host cell transformed with an expression vector that contains a gene encoding IFN-β. The host cell is one that can transcribe the gene and produce the desired protein. These techniques have been used to produce IFN-β in bacterial (Taniguchi, T. et al. (1980b) Proc. Natl. Acad. Sci. USA 77:5230-5233; Derynck, R. et al. (1980) Nature 287:193-197; and Goeddel, D.V. et al. (198) Nucl. Acids Res. 8:4057-4074), mammalian (Ohno, S. and Taniguchi, T. (1982) Nucl. Acids Res. 10:967-977; and McCormick, F. et al. (1984) Mol. Cell Biol. 4:166-172), and insect (Smith, G.E. et al. (1983) Mol. Cell. Biol. 3:2156-2165) host cells.

Muteins of IFN-β having improved characteristics have been produced, as described in, for example, Mark, D.F. et al. (1984) Proc. Natl. Acad. Sci. USA 81:5662-5666 and U.S. Patent No. 4,588,585. Muteins are mutationally altered biologically active proteins or polypeptides that are produced by directed mutagenesis techniques. IFN-β-1 b is a mutein of IFN-β in which Cys17 is changed to Ser17 *via* a T to A transition in the first base of codon 17, which prevents incorrect disulfide bond formation. The production and purification of IFN-β-1b expressed in *E. coli* is described in, for example, Lin, L.S. et al. (1986) Meth. Enzymol. 119:183-192 and U.S. Patent No. 5,814,485.

Two different forms of IFN-β have been approved and are available for the treatment of relapsing remitting MS: (1) IFN-β-1a, which includes Avonex^{®} and Rebif^{®}; and (2) IFN-β-1b, which includes Betaseron^{®} and Betaferon^{®}. These marketed IFN-β products decrease the relapse rate, slow disease progression, and improve inflammatory measures of disease activity. See, for example, Vartanian, T. (2003) Clin. Ther. 25:105-118.

IFN-β-1 a differs from IFN-β-1b in several ways: (1) IFN-β-1a is a N-linked glycosylated protein that is expressed in Chinese hamster ovary ("CHO") cells, whereas IFN-β-1b is a non-glycosylated protein that is expressed in *E. coli;* (2) IFN-β-1a is 166 amino acids in length with Met1 at the N-terminus, whereas when IFN-β-1b is processed in E.coli and the N-terminal methionine is removed, it is 165 amino acids in length with Ser2 at the N-terminus; (3) like native IFN-β, IFN-β-1a has a cysteine residue at position 17, whereas in IFN-β-1b Cys17 is replaced with Ser17; and (4) IFN-β-1a has a specific activity approximately 10-fold higher than IFN-β-1b. See, for example, Runkel, L. et al. (1998) Pharm. Res. 15:641-649; Weber, F. et al. (1999) Neurology 52:1069-1071; and Francis, G. et al. (2000) Neurology 55:322-323.

Gangliosides are sialic acid-containing glycosphingolipids that are occur ubiquitously in vertebrate cell plasma membranes and are particularly abundant in the nervous system. See, for example, Lloyd, K.O. and Furukawa, K. (1998) Glycocon. J. 15:627-636. Gangliosides are involved in the control of cell growth and differentiation, which are mediated through cell-cell recognition or cell-matrix interaction *via* lectin-like proteins or by carbohydrate-carbohydrate interactions. Gangliosides consist of a lipid moiety linked to a large family of oligosaccharide structures that differ in glycosidic linkage position, sugar conformation, neutral sugar, and sialic acid content. The ganglioside profile of human serum consists of at least ten different compounds, including, in decreasing order in abundance, GM₃, GD₃, GD₁ₐ, GM₂, GT_{1b}, MG-3, MG-4, and GD_{1b}, as described in Senn, H.J. et al. (1989) Eur. J. Biochem. 181:657-662.

Gangliosides have been shown to block the specific activity of certain human IFNs (also called herein "ganglioside inhibitory activity"), e.g., IFN antiviral activity, under particular experimental conditions. For example, the antiviral activity of fibroblast-derived native IFN-β, but not leukocyte-derived native IFN-α, is inhibited by pre-incubation with mixed gangliosides (Gupta, S.L. et al. (1984) J. Interferon Res. 4:305-314), and the antiviral activity of native IFN-β is abolished by the gangliosides GM₁, GM₂, and GM₃, but not by sialic acid (*N*-acetylneuraminic acid) alone (Fuse, A. et al. (1982) Antiviral Res. 2:161-166). These results, however, were obtained with only partially purified native interferons and not with more highly purified recombinant IFN-β-1a or IFN-β-1b.

### SUMMARY OF THE INVENTION

Described herein are compositions comprising an improved IFN-β-1b polypeptide, or fragment, analog, or variant thereof, that has a greater specific activity than a reference IFN-β composition, and methods of producing such IFN-β-1b compositions. Examples of reference IFN-β compositions include, e.g., a reference IFN-β-1b composition or a reference IFN-β-1 a composition.

Gangliosides are known to inhibit IFN-β specific activity (also called herein "ganglioside inhibitory activity"). Thus, in some aspects, the IFN-β-1b compositions have decreased ganglioside inhibitory activity. For example, described herein are pharmaceutical compositions, comprising an IFN-β 1 b polypeptide, or fragment, analog, or variant thereof, that have decreased ganglioside inhibitory activity and have greater IFN-β specific activity as compared to a reference IFN-β composition. In one aspect, the pharmaceutical compositions are substantially free of gangliosides. Further, in some aspects, the IFN-β specific activity of a pharmaceutical composition of the present invention is at least 2-fold, at least 4-fold, at least 6-fold, at least 8-fold, or at least 10-fold greater than a reference IFN-β composition.

The present invention provides a process comprising decreasing the ganglioside inhibitory activity of a composition comprising an IFN-β-1b composition, to obtain a composition that has decreased ganglioside inhibitory activity and has a greater IFN-β specific activity than a reference IFN-β composition wherein said decreasing of said ganglioside inhibitory activity is by selective oxidation of said IFN-β-1b polypeptide. In one aspect, the process comprises a step of removing gangliosides from the composition such that the composition is substantially free of gangliosides. In a further aspect, the ganglioside inhibitory activity is decreased by selectively oxidizing the composition, e.g., by heat, chemical, or enzymatic oxidation. In one aspect, the oxidation is by an oxidizing agent. In preferred aspects, the oxidizing agent is selected from a group consisting of sodium periodate, o-iodosobenzoic acid, CuCl₂, and o-phenanthroline. In one preferred aspect, the oxidizing agent is sodium periodate. Also described are pharmaceutical compositions, comprising an IFN-β-1b, obtained from such a process, where the pharmaceutical compositions have a decreased ganglioside inhibitory activity and have an IFN-β specific activity that is greater than a reference IFN-β composition. In one aspect, the pharmaceutical composition obtained from such a process is substantially free of gangliosides.

Also described herein are improved IFN-β-1b polypeptides, or fragments, analogs, derivatives, or variants thereof, that are selectively oxidized and have greater specific activity as compared to a reference IFN-β composition. Further described are pharmaceutical compositions comprising such improved IFN-β-1b polypeptides, or fragments, analogs, derivative, or variants thereof. In some aspects, the specific activity of an improved IFN-β-1b polypeptide, or fragment, analog, derivative, or variant thereof, of the present invention is at least 2-fold, at least 4-fold, at least 6-fold, at least 8-fold, or at least 10-fold greater than a reference IFN-β composition. In some aspects the selective oxidation is by heat, chemical, or enzymatic oxidation. In one aspect, the oxidation is by an oxidizing agent. In preferred aspects, the oxidizing agent is selected from a group consisting of sodium periodate, o-iodosobenzoic acid, CuCl₂, and o-phenanthroline. In one preferred aspect, the oxidizing agent is sodium periodate.

The present invention also provides a process comprising selectively oxidizing an IFN-β-1b polypeptide to obtain an improved IFN-β-1b polypeptide, wherein the improved IFN-β-1b polypeptide has a greater specific activity than a reference IFN-β composition. In some aspects the selective oxidation is by heat, chemical, or enzymatic oxidation. In one aspect, the oxidation is by an oxidizing agent. In preferred aspects, the oxidizing agent is selected from a group consisting of sodium periodate, o-iodosobenzoic acid, CuCl₂, and o-phenanthroline. In one preferred aspect, the oxidizing agent is sodium periodate. The present invention also provides improved IFN-β-1b polypeptides, and pharmaceutical compositions comprising improved IFN-β-1b polypeptides, obtained by such a process.

Described herein are *in vitro* and *in vivo* assays to characterize the improved IFN-β-1b polypeptides and pharmaceutical compositions .

Further described are compositions comprising a pharmaceutically acceptable carrier and an improved IFN-β-1b polypeptide or pharmaceutical composition .

Methods for using the IFN-β-1b compositions are provided. The IFN-β-1b compositions are useful for treating multiple sclerosis, e.g., relapsing remitting multiple sclerosis ("MS"), as well as other forms of MS, including secondary progressive MS, primary progressive MS, and progressive relapsing MS.

### BRIEF DESCRIPTION OF THE FIGURES

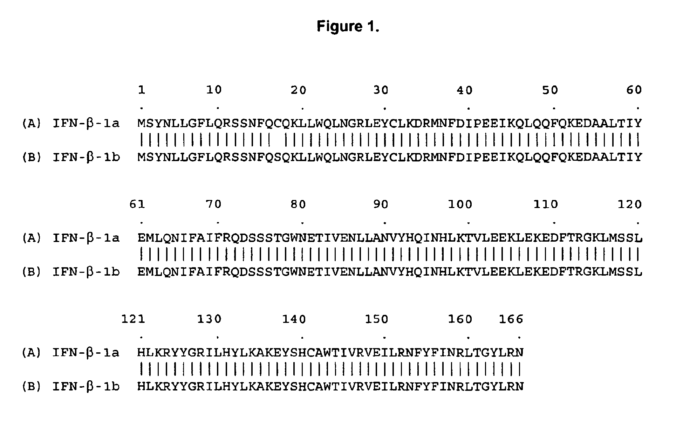

**Figure 1****.** Amino acid sequences of IFN-β-1a (SEQ ID NO: 1) and IFN-β-1b (SEQ ID NO: 2). The polynucleotide sequences, amino acid sequences, and structure of IFN-β-1a are described in, for example, Innis, M.A. and McCormick, F. (1986) Meth. Enzymol. 119:397-403 and U.S. Patent No. 4,966,843. The polynucleotide sequences, amino acid sequences, and structure of IFN-β-1b are described in, for example, Mark, D.F. *et al.* (1984) *supra* and U.S. Patent No. 4,588,585. When IFN-β-1b is processed in E.coli and the N-terminal methionine is removed, it is 165 amino acids in length with Ser2 at the N-terminus.

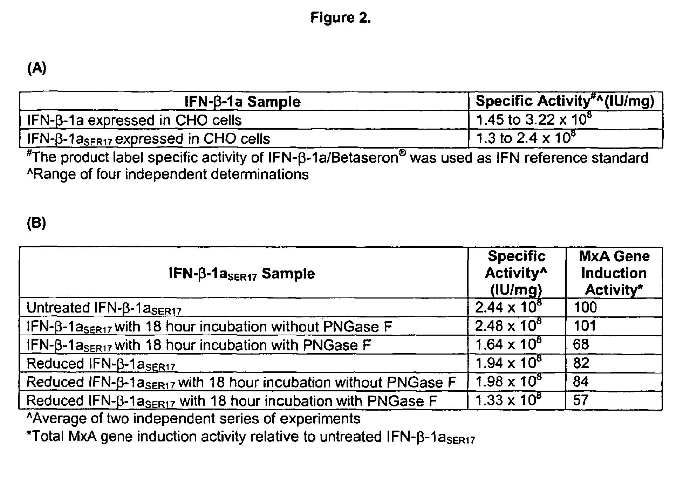

**Figure 2****.** Effect of N-terminal Met1, Cys17 replacement by Ser17, N-linked glycosylation, and SDS on the biological activity of IFN-β-1a *in vitro.* (A) IFN-β-1a and IFN-β-1a_{SER17}, in which Cys17 is replaced by Ser17, were expressed in CHO cells, purified by affinity chromatography, and tested in the MxA gene induction assay, as described in Example 1. (B) Reduced and non-reduced IFN-β-1a_{SER17} were deglycosylated with peptide N-glycosidase F ("PNGase F") and tested in the MxA gene induction assay.

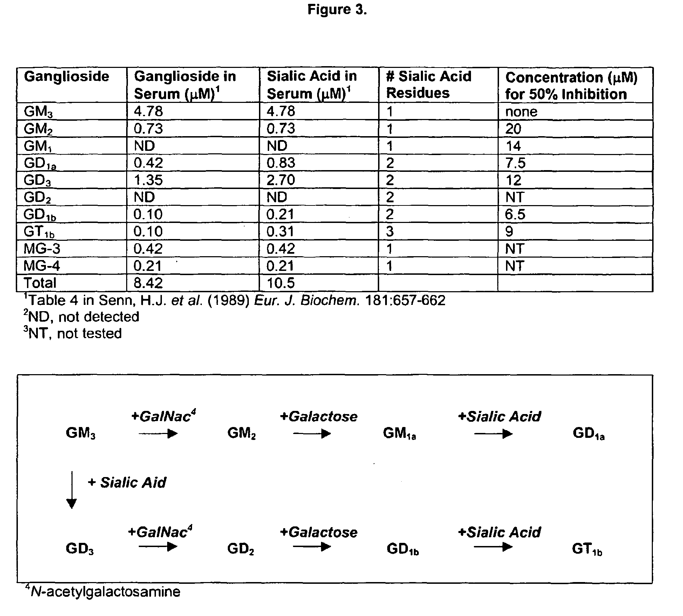

**Figure 3****.** Effect of gangliosides on the biological activity of IFN-β-1b *in vitro.* IFN-β-1b was exposed to various concentrations of the indicated gangliosides, and then tested in the MxA gene induction assay *in vitro,* as described in Example 2. Pathways leading to the generation of the various gangliosides starting from GM₃ are indicated.

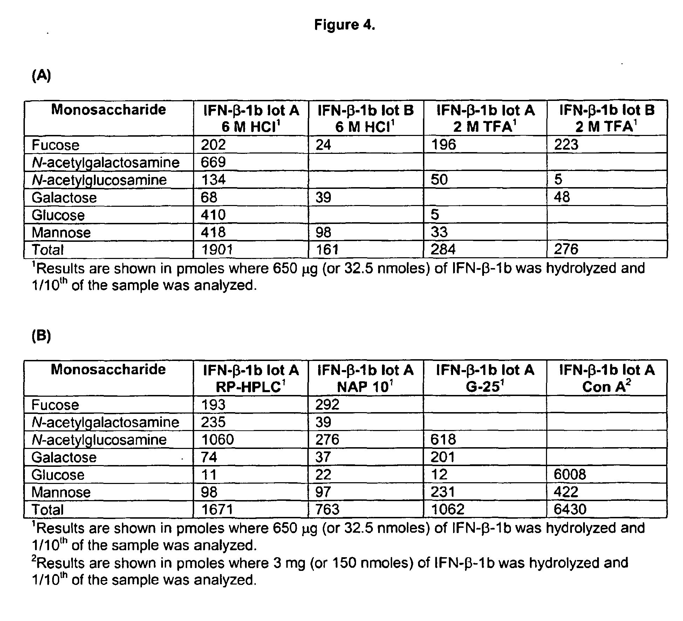

**Figure 4****.** Presence of carbohydrate in IFN-β-1b preparations. Two lots of IFN-β-1b were analyzed for monosaccharide content (A) before and (B) after further purification. (A) IFN-β-1 b lots A and B were subjected to acid hydrolysis in 6 M HCl or 2 M TFA and analyzed for the presence of six monosaccharides, as described in Example 3. (B) IFN-β-1b lot A was further purified by either RP-HPLC, NAP 10, Sephadex G-25, or Con A Sepharose column chromatography as indicated, and then analyzed for the presence of six monosaccharides.

### DETAILED DESCRIPTION OF THE INVENTION

The pharmaceutical compositions prepared by a process of the present invention comprise an IFN-β-1b polypeptide, fragment, analog, derivative, or variant thereof, and have an improved IFN-β-1b specific activity. The improved IFN-β-1b polypeptides, or fragments, analogs, derivatives, or variants thereof, are comprised of IFN-β-1b polypeptides that have a greater specific activity as compared to a reference IFN-β composition. In particular embodiments, the pharmaceutical compositions , comprising an IFN-β-1b, have decreased ganglioside inhibitory activity and a greater IFN-β specific activity. In one aspect, the pharmaceutical compositions are substantially free of gangliosides which inhibit IFN-β specific activity, and have an improved IFN-β specific activity. Methods are provided for obtaining the improved IFN-β-1b compositions. In a preferred embodiment, the improved IFN-β-1b compositions are obtained by selectively oxidizing the IFN-β-1b polypeptide.

The present invention provides processes for obtaining IFN-β-1b compositions having improved specific activity. The processes of the present invention are useful for decreasing or inactivating gangliocide inhibitory activity and/or for removing (or reducing the amount of) gangliosides which can inhibit IFN-β specific activity and, consequently, the IFN-β-1b compositions of the present invention have an improved IFN-β specific activity.

### Definitions

In describing the present invention, the following terms are defined as indicated below.

"Recombinant proteins or polypeptides" refer to proteins or polypeptides produced by recombinant DNA techniques, *i.e*., produced from cells, microbial or mammalian, transformed by an exogenous recombinant DNA expression construct encoding the desired protein or polypeptide. Proteins or polypeptides expressed in most bacterial cultures will typically be free of glycan. Proteins or polypeptides expressed in yeast may have a glycosylation pattern different from that expressed in mammalian cells.

"Native" or "naturally occurring" proteins or polypeptides refer to proteins or polypeptides recovered from a source occurring in nature. The term "native IFN-β" or "naturally occurring IFN-β" would include native or naturally occurring IFN-β and fragments thereof, and would include post-translational modifications of IFN-β and fragments thereof, including, but not limited to, acetylation, carboxylation, glycosylation, phosphorylation, lipidation, acylation, and cleavage.

A DNA or polynucleotide "coding sequence" is a DNA or polynucleotide sequence that is transcribed into mRNA and translated into a polypeptide in a host cell when placed under the control of appropriate regulatory sequences. The boundaries of the coding sequence are the start codon at the 5' N-terminus and the translation stop codon at the 3' C-terminus. A coding sequence can include prokaryotic sequences, cDNA from eukaryotic mRNA, genomic DNA sequences from eukaryotic DNA, and synthetic DNA sequences. A transcription termination sequence will usually be located 3' to the coding sequence.

"DNA or polynucleotide sequence" is a heteropolymer of deoxyribonucleotides (bases adenine, guanine, thymine, cytosine). DNA or polynucleotide sequences encoding the proteins or polypeptides can be assembled from synthetic cDNA-derived DNA fragments and short oligonucleotide linkers to provide a synthetic gene that is capable of being expressed in a recombinant DNA expression vector. In discussing the structure of particular double-stranded DNA molecules, sequences may be described herein according to the normal convention of providing only the sequence in the 5' to 3' direction along the non-transcribed strand of cDNA.

"Recombinant expression vector or plasmid" is a replicable DNA vector or plasmid construct used either to amplify or to express DNA encoding the proteins or polypeptides . An expression vector or plasmid contains DNA control sequences and a coding sequence. DNA control sequences include promoter sequences, ribosome binding sites, polyadenylation signals, transcription termination sequences, upstream regulatory domains, and enhancers. Recombinant expression systems as defined herein will express the proteins or polypeptides upon induction of the regulatory elements.

"Transformed host cells" refer to cells that have been transformed and transfected with exogenous DNA. Exogenous DNA may or may not be integrated (i.e., covalently linked) to chromosomal DNA making up the genome of the host cell. In prokaryotes and yeast, for example, the exogenous DNA may be maintained on an episomal element, such as a plasmid, or stably integrated into chromosomal DNA. With respect to eukaryotic cells, a stably transformed cell is one which is the exogenous DNA has become integrated into the chromosome. This stability is demonstrated by the ability of the eukaryotic cell lines or clones to produce *via* replication a population of daughter cells containing the exogenous DNA.

"IFN or IFNs" refers to the family of secreted proteins known as interferons, which are cytokines with antiviral, anti-protozoal, immunomodulatory, and cell growth regulatory activities. IFNs were originally classified by their sources: leukocytes IFN-α-1 and IFN-α-2), fibroblasts (IFN-β), and immune cells (IFN-γ). See, for example, Peskta, S. (1986) *supra;* Sen, G.C. and Lengyel, P. (1992) *supra;* and Pestka, S., ed., Interferons Part C in Meth. Enzymol. Vol. 119, Academic Press, Inc., New York, NY. (1986).

"IFN-β" refers to fibroblast IFN, which in man is a single gene lacking introns. The DNA or polynucleotide sequence of human IFN-β is described in Taniguchi, T. *et al.* (1980a) *supra* and U.S. Patent No. 5,326,859. The human IFN-β cDNA encodes a pro-polypeptide 187 amino acids in length. A 21 amino acid signal sequence is cleaved off to form the mature, secreted IFN-β polypeptide, which is 166 amino acids in length.

"IFN-β-1a" refers to recombinant human IFN-β expressed in Chinese hamster ovary ("CHO") cells. As shown in Figure 1, mature secreted IFN-β-1a is 166 amino acids in length, corresponding to native IFN-β. IFN-β-1a is N-linked glycosylated at the asparagine residue at position 80 (Asp80). See, for example, Innis, M.A. and McCormick, F. *et al.* (1986) *supra* and U.S. Patent No. 4,966,843.

"IFN-β-1b" refers to recombinant human IFN-β expressed in *E. coli* host cells and having a cysteine to serine amino acid substitution at position 17 (Ser17). When IFN-β-1b is processed in E.coli and the N-terminal methionine is removed, it is 165 amino acids in length with Ser2 at the N-terminus. IFN-β-1b is not glycosylated. See, for example, Mark, D.F. *et al.* (1984) *supra* and U.S. Patent No. 4,588,585.

"Improved IFN-β-1b" compositions are IFN-β-1b compositions (e.g., IFN-β-1b polypeptides, or fragments, analogs, derivatives, or variants thereof, and pharmaceutical composition comprising IFN-β-1b polypeptides, or fragments, analogs, derivatives, or variants thereof) having a greater specific activity as compared to a reference IFN-β composition. In a preferred embodiment, the improved IFN-β-1b compositions prepared by a process of the present invention are obtained by selectively oxidizing the IFN-β-1b composition using heat, chemical, or enzymatic oxidation. In another preferred embodiment, the improved IFN-β-1b compositions are obtained by selectively oxidizing the IFN-β-1b composition using an oxidizing agent. In another preferred embodiment, an IFN-β-1b composition prepared by a process of the present invention is obtained by decreasing the amount of ganglioside inhibitory activity in the composition. In another preferred embodiment, an IFN-β-1b composition prepared by a process of the present invention is obtained by removing gangliosides from the composition such that the composition is substantially free of gangliosides.

A "selectively oxidized IFN-β-1b polypeptide, or fragment, analog, or variant thereof" is an improved IFN-β-1b composition wherein an IFN-β-1b polypeptide, or fragment, analog, or variant thereof, is selectively oxidized as described herein to obtain an improved IFN-β-1b polypeptide (or improved IFN-β-1b composition) having a greater specific activity than a reference IFN-β composition.

"Substantially free of gangliosides" with reference to the IFN-β-1b compositions prepared by a process of the present invention means that the gangliosides in the IFN-β-1b composition are not detectable by the methods described herein or known methods of detection, or that the inhibitory activity of the gangliosides is decreased in the composition as compared to a reference IFN-β composition. In preferred embodiments, the IFN-β-1b compositions prepared by a process of the present invention are obtained using the processes described herein to remove (or decrease the amount of) gangliosides, and/or to decrease or inactivate ganglioside inhibitory activity, in the IFN-β-1b compositions. Thereby, IFN-β-1b compositions are obtained that are substantially free of gangliosides and/or have decreased or inactivated ganglioside inhibitory activity.

The terms "analog", "fragments", "derivative", and "variant", when referring to the improved IFN-β-1b polypeptides mean analogs, fragments, derivatives, and variants of such improved IFN-β-1b polypeptides that retain substantially similar functional activity or substantially the same biological function or activity as the improved IFN-β-1b polypeptides, as described herein.

An "analog" includes a pro-polypeptide that includes within it, the amino acid sequence of an improved IFN-β-1b polypeptide . The improved IFN-β-1b polypeptide can be cleaved from the additional amino acids that complete the pro-IFN-β-1b molecule by natural, *in vivo* processes or by procedures well known in the art, such as by enzymatic or chemical cleavage. For example, IFN-β-1b (see Figure 1) is expressed in *E. coli* as a 166 amino acid pro-IFN-β-1b, which is processed *in vivo* to release the 165 amino acid active, mature IFN-β-1b.

A "fragment" is a portion of an improved IFN-β-1b polypeptide that retains substantially similar functional activity or substantially the same biological function or activity as an improved IFN-β-1b polypeptide, as shown in the *in vitro* assays disclosed herein, as described further below.

A "derivative" includes all modifications to an improved IFN-β-1b polypeptide of this invention that substantially preserve the functions disclosed herein and include additional structure and attendant function, *e.g*., PEGylated polypeptides, which have greater half-life.

A "variant" includes polypeptides having an amino acid sequence sufficiently similar to the amino acid sequence of the original improved IFN-β-1b polypeptides. The term "sufficiently similar' means a first amino acid sequence that contains a sufficient or minimum number of identical or equivalent amino acid residues relative to a second amino acid sequence such that the first and second amino acid sequences have a common structural domain and/or common functional activity. For example, amino acid sequences that contain a common structural domain that is at least about 45%, preferably about 75% through 98%, identical are defined herein as sufficiently similar. Preferably, variants will be sufficiently similar to the amino acid sequence of the preferred improved IFN-β-1b polypeptides . Variants include variants of improved IFN-β-1b polypeptides encoded by a polynucleotide that hybridizes to a polynucleotide , or a complement thereof, under stringent conditions. Such variants generally retain the functional activity of the improved IFN-β-1 b polypeptides . Variants include improved IFN-β-1b polypeptides that differ in amino acid sequence due to mutagenesis. Variants that have antiviral activity can be identified by screening combinatorial libraries of mutants, for example truncation or point mutants, of improved IFN-β-1b polypeptides using the MxA gene induction assay, which is described under Assays to Measure the Specific Activity of IFN-β-1b Polypeptides.

"Substantially similar functional activity" and "substantially the same biological function or activity" each means that the degree of biological activity is within about 50% to 100% or more, preferably within 80% to 100% or more, and more preferably within about 90% to 100% or more, of that biological activity demonstrated by the polypeptide to which it is being compared when the biological activity of each polypeptide is determined by the same procedure or assay. For example, a polypeptide that has substantially similar functional activity to IFN-β-1b is one that, after oxidation in the assays described in Example 4, demonstrates the ability to induce the MxA gene or to inhibit virus production *in vitro,* as described under Assays to Measure the Specific Activity of IFN-β-1b Polypeptides.

"Similarity" between two polypeptides is determined by comparing the amino acid sequence of one polypeptide to the sequence of a second polypeptide. An amino acid of one polypeptide is similar to the corresponding amino acid of a second polypeptide if it is identical or a conservative amino acid substitution. Conservative substitutions include those described in Dayhoff, M.O., ed., The Atlas of Protein Sequence and Structure 5, National Biomedical Research Foundation, Washington, D.C. (1978), and in Argos, P. (1989) EMBO J. 8:779-785. For example, amino acids belonging to one of the following groups represent conservative changes or substitutions:
- Ala, Pro, Gly, Gln, Asn, Ser, Thr:
   - Cys, Ser, Tyr, Thr;
   - Val, Ile, Leu, Met, Ala, Phe;
   - Lys, Arg, His;
   - Phe, Tyr, Trp, His; and
   - Asp, Glu.

"Mammal" includes humans and domesticated animals, such as cats, dogs, swine, cattle, sheep, goats, horses, rabbits, and the like.

"Specific activity" or "IFN-β specific activity" as used herein with reference to an IFN-β-1b composition prepared by a process of the present invention means a biological activity or function of an IFN-β, e.g., IFN-β-1b or IFN-β-1a. The biological activities or functions of IFN-β-1bb and IFN-β-1a are well-known in the art and include, but are not limited to, e.g., antiviral activity. Such specific activity can be detected and measured using the methods described herein or using any method known in the art (see *e.g*., assays described in Fellous et al. (1982) Proc. Natl. Acad. Sci USA 79:3082-3086; Czerniecki et al. (1984) J. Virol. 49(2):490-496; Mark et al. (1984) Proc. Natl Acad. Sci. USA 81:5662-5666; Branca et al. (1981) Nature 277:221-223; Williams et al. (1979) Nature 282:582-586; Herberman et al. (1979) Nature 277:221-223; Anderson et al. (1982) J. Biol. Chem. 257(19):11301-11304).

In a preferred embodiment, the IFN-β-1b composition prepared by a process of the present invention has a specific activity as detected and measured using the MxA gene induction assay or the viral production assay, as described herein under Assays to Measure the Specific Activity of IFN-β-1 b Polypeptides. Antiviral activity is determined relative to the activity of a reference human IFN-β, e.g., reference IFN-β-1b or IFN-β-1a (see, e.g., Pestka, S. (1986) Meth. Enzymol. 199:14-23). In one embodiment, the specific activity of the IFN-β-1b used herein is ∼3 x 10⁷ IU/mg, as determined using the antiviral cytopathic effect ("CPE") assay with A549 cells and murine encephalomyelitis ("EMC") virus.

"IFN-β-1b composition" refers to an IFN-β-1b polypeptide, fragment, analog, derivative, or variant thereof, or composition (e.g., a pharmaceutical composition) comprising an IFN-β-1b polypeptide, fragment, analog, derivative, or variant thereof.

"Improved specific activity" as used herein means that the specific activity of the IFN-β-1b composition prepared by a process of the present invention is greater than that of a reference IFN-β composition. Suitable reference IFN-β compositions include, e.g., reference IFN-β-1b compositions, or reference IFN-β-1a compositions. For example, in one embodiment, the IFN-β-1b composition prepared by a process of the present invention has a specific activity, as measured in the MxA gene induction assay or the viral production assay, as described herein under Assays to Measure the Specific Activity of IFN-β-1b Polypeptides, that is greater than the specific activity of a reference IFN-β-1b composition. An improved specific activity of an IFN-β-1b composition prepared by a process of the present invention is preferably at least 2-fold, at least 4-fold, at least 6-fold, at least 8-fold, or at least 10-fold greater than the specific activity of a reference IFN-β composition (e.g., a reference IFN-β-1b composition or a reference IFN-β-1a composition).

Gangliosides can inhibit IFN-β specific activity (also called herein "ganglioside inhibitory activity"). The present invention provides processes for decreasing ganglioside inhibitory activity or removing gangliosides from the IFN-β-1b compositions and, consequently, increasing IFN-β specific activity. Gangliosides can be removed or decreased in a composition using the methods described herein or known in the art. Similarly, ganglioside inhibitory activity can be decreased or diminshed as described herein or using methods known in the art e.g., to remove, decrease, or inactivate gangliosldes.

"Reference *IFN-β composition" refers to a composition comprising an IFN-β (e.g., IFN-β-1b or IFN-β-1a) that is not treated or manipulated as described herein to improve an IFN-β specific activity thereof and/or as described herein for obtaining an IFN-β-1b composition of the present invention. The reference IFN-β composition may be used as a standard to compare and determine the relative specific activity of the reference IFN-β composition to the specific activity of an IFN-β-1b composition of the present invention. For example, in one embodiment, the IFN-β of the reference composition is not selectively oxidized, whereas the improved IFN-β-1b polypeptide is obtained by selective oxidation of the IFN-β-1b polypeptide and, thereby, resulting in an IFN-β-1b composition that has greater specific activity as compared to the reference IFN-β composition.

Also for example, in another embodiment, the IFN-β of the reference composition is not treated to remove (or reduce the amount of) gangliosides, whereas the IFN-β-1b composition of the present invention is treated to remove (or to reduce the amount of) gangliosides resulting in an IFN-β-1b composition that is substantially free of gangliosides as compared to the reference IFN-β composition. Also for example, in another embodiment, the IFN-β of the reference composition is not treated to decrease or inactivate ganglioside inhibitory activity, whereas the IFN-β-1b composition of the present invention is treated to decrease or inactivate ganglioside inhibitory activity resulting in an IFN-β-1b composition that has increased specific activity as compared to the reference IFN-β composition.

Methods of the present invention for removing (or reducing the amount of) gangliosides, or for decreasing or inactivating ganglioside inhibitory activity, are described herein, and include but are not limited to, e.g., selective oxidation of the IFN-β-1b polypeptide. Methods of the present invention for selectively oxidizing IFN-β-1b polypeptides include but are not limited to, e.g., using heat, chemical, or enzymatic oxidation.

The specific activity of an IFN-β-1b composition of the present invention can be assayed as compared to the specific activity of the reference IFN-β composition, using a method for detecting and/or measuring an IFN-β specific activity, e.g., as described herein or as known in the art.

"Therapeutically effective amount" refers to that amount of an improved IFN-β-1b polypeptide of the invention which, when administered to a human in need thereof, is sufficient to effect treatment, as defined below, for relapsing remitting multiple sclerosis ("MS"), as well as other forms of MS, including secondary progressive MS, primary progressive MS, and progressive relapsing MS. The amount of an improved IFN-β-1b polypeptide of the invention which constitutes a "therapeutically effective amount" will vary depending on the IFN-β-1b protein, the condition and its severity, and the age of the human to be treated, but can be determined routinely by one of ordinary skill in the art having regard to his own knowledge and to this disclosure.

"Treating" or "treatment" as used herein covers the treatment of disease-state in a mammal, preferably a human, which disease-state is characterized by symptoms associated with MS, such as weakness, numbness, tremor, loss of vision, pain, paralysis, loss of balance, bladder and bowel dysfunction, and cognitive changes (primary symptoms); repeated urinary tract infections, disuse weakness, poor postural alignment and trunk control, muscle imbalance, decreased bone density, shallow, inefficient breathing, and bedsores (secondary symptoms); and depression (tertiary symptoms), and includes:
(i) inhibiting the condition, *i.e*., arresting its development; or
(ii) relieving the condition, *i.e*., causing regression of the condition.

All other technical terms used herein have the same meaning as is commonly used by those skilled in the art to which the present invention belongs.

### Characteristics of the Improved IFN-β-1b Polypeptides of the Invention

The improved IFN-β-1b compositions prepared by a process of the present invention comprise IFN-β-1b polypeptides, or fragments, analogs, derivatives, or variants thereof, that have at least 2-fold, preferably at least 4-fold, more preferably at least 6-fold, even more preferably 8-fold, and even more preferably at least 10-fold, greater specific activity than that of a reference IFN-β composition. In preferred embodiments, the reference IFN-β composition is a reference IFN-β-1b composition. In some embodiments, the reference IFN-β composition is a reference IFN-β-1a composition. In one embodiment, the improved IFN-β-1b compositions of the present invention have at least 2-fold greater specific activity than a reference IFN-β-1b composition expressed in *E. coli* and purified according to the procedure described in U.S. Patent No. 5,814,485.

### Expression and Purification of Improved IFN-β-1b Polypeptides

There are several ways to express and purify recombinant human IFN-β-1b in bacteria, in particular in *E. coli,* to obtain IFN-β-1b polypeptides having improved specific activity. Known methods can be used to express the cloned genes in bacteria. To obtain high-level expression of a cloned eukaryotic gene in a prokaryotic system, it is preferable to construct expression vectors that contain, a strong promoter to direct mRNA transcription. Examples of regulatory regions suitable for this purpose are the promoter and operator region of the *E. coli* beta-glucosidase gene, the *E*. *coli* tryptophan biosynthetic pathway, or the leftward promoter from phage λ. The inclusion of selection markers in DNA plasmids transformed in *E*. *coli* is also useful. Examples of such markers include the genes specifying resistance to ampicillin, tetracycline, or chloramphenicol.

Post-translational modifications, such as glycosylation, do not occur in the prokaryotic cell expression system *E. coli.* In addition, proteins with complex disulfide patterns are can be misfolded when expressed in *E. coli.* With the prokaryotic system, the expressed protein is either present in the cell cytoplasm in an insoluble form, in so-called inclusion bodies, found in the soluble fraction after the cell has lysed, or is directed into the periplasm by the addition of appropriate secretion signal sequences. If the expressed protein is in insoluble inclusion bodies, solubilization and subsequent refolding of the inclusion bodies are usually required.

Many prokaryotic expression vectors are known to those of skill in the art and are commercially available, such as pKK223-3 (Pharmacia Fine Chemicals, Uppsala, Sweden), pKK233-2 (Clontech, Palo Alto, CA, USA), and pGEM1 (Promega Biotech, Madison, WI, USA).

Promoters commonly used in recombinant microbial expression systems include the beta-lactamase (penicillinase) and lactose promoter system (Chang, A.C. et al. (1978) Nature 275:617-624; and Goeddel, D.V. et al. (1979) Nature 281:544-548), the tryptophan (trp) promoter system (Goeddel, D.V. *et al.* (1980) *supra*), and the tac promoter (Sambrook, J.F. et al., Molecular Cloning: A Laboratory Manual, Cold Spring Harbor Laboratory, Cold Spring Harbor, N.Y. (1989)). Another useful bacterial expression system employs the lambda phage pL promoter and clts857 thermoinducible repressor (Bernard, H.U. et al. (1979) Gene 5:59-76; and Love, C.A. et al. (1996) Gene 176:49-53).

The process of manufacturing IFN-β-1b/Betaseron^{®} and its subsequent analysis and release testing are reviewed in Lin, L. (1998) Dev. Biol. Stand. 96:97-104. IFN-β-1b/Betaseron^{®} is a form of IFN-β-1b expressed in *E. coli* under control of the trp promoter, and produced and purified as described in U.S. Patent No. 5,814,485, the disclosure of which is incorporated by reference in its entirety. In one embodiment, the specific activity of such IFN-β-1b preparations is ∼3 x 10⁷ IU/mg.

A preferred method to express and purify the improved IFN-β-1b polypeptides is the process described in U.S. Patent No. 5,814,485.

### Methods of Oxidation of Improved FN-β-1b Polypeptides

The oxidation of recombinant mammalian proteins expressed in bacteria, such as IFNs, is typically employed for the formation of disulfide bridging that is identical to their naturally occurring counterparts. Reagents useful for oxidizing reduced, cysteine-containing bacterially expressed mammalian proteins include *o*-iodosobenzoic acid, CuCl₂, and *o-*phenanthroline, as described in U.S. Patent Nos. 4,530,787 and 4,572,798.

Gangliosides can inhibit the specific activity of IFN-β-1b (also called herein "ganglioside inhibitory activity"). An object of the present invention is that oxidation is also useful for the oxidative cleavage of carbohydrates that are present in highly purified preparations of *E*. *coli* expressed mammalian proteins, such as IFN-β-1b. Such processes of the present invention are useful for removing (or reducing the amount of) gangliosides in an IFN-β-1b composition, and/or for reducing or inactivating the inhibitory activity of gangliosides and consequently increasing the specific activity of the IFN-β-1b compositions of the present invention.

Methods to oxidize or cleave carbohydrates are well known and include, for example, acid hydrolysis, enzymatic hydrolysis, periodate oxidation, and heating. Periodate oxidation is used to oxidize monosaccharides, polysaccharides, and oligosaccharides (see, for example, Pappas, R.S. et al. (1990) Carbohydr. Res. 197:1-14 and Sussich, F. and Cesaro, A. (2000) Carbohydr. Res. 329:87-95), and proteins and peptides (see, for example, Clamp, J.R. and Hough, L. (1965) Biochem. J. 94:17-24).

### Periodate Oxidation

Rapid oxidation by periodate of proteins and peptides generates an aldehyde in the peptide and is used as the first step in a method for site-directed labeling with, for example, biotin, a fluorescent reporters, metal chelating groups, imaging agents, cytotoxic drugs, or stabilizing agents, such as polyethylene glycol (Geoghegan, K.F. and Stroh, J.G. (1992) Bioconjugate Chem. 3:138-146). The target for periodate oxidation is the 2-amino alcohol structure, -CH(NH₂)CH(OH)-, which exists in proteins and peptides in N-terminal serine or threonine residues and in hydroxylysine residues.

Periodate can also oxidize several amino acid residues in proteins, including tyrosine, methionine, histidine, and tryptophan (Clamp, J.R. and Hough, L. (1965) *supra).* Conditions for the selective oxidation of N-terminal serine or threonine, with minimal oxidation of other amino acid residues, have been described in, for example, Geoghegan, K.F. and Stroh, J.G. (1992) *supra;* and Gaertner, H.F. and Offord, R.E. (1996) Bioconjugate Res. 7:38-44, the disclosures of which are incorporated in full by reference. Three examples of recombinant mammalian cytokine proteins that have been selectively oxidized at their N-terminus include: (1) interleukin-1α ("IL-1α"); (2) interleukin-8 ("IL-8"); and (3) granulocyte-colony stimulating factor ("G-CSF").
(1) The N-terminal serine in IL-1α (13 µM) was oxidized with 40 µM sodium periodate (3-fold molar excess) in 0.2 M sodium phosphate, pH 7.0, for 30 minutes at 0°C. Periodate was removed by gel filtration using Sephadex G-25 equilibrated with 50 mM sodium acetate, pH 4.5. Quantitative oxidation of the N-terminus was confirmed by isoelectric focussing. See Geoghegan, K.F. and Stroh, J.G. (1992) *supra.*
(2) The N-terminal serine in IL-8 (at 2 to 5 mg/ml) was oxidized with a 10-fold excess of sodium periodate in 1 % NH₄-HCO₃ buffer, pH 8.3, in the presence of a 50-fold molar excess of methionine for 10 minutes at room temperature. The reaction was stopped by the addition of a 2000-fold molar excess of ethylene glycol over periodate, and the solution further incubated for 15 minutes at room temperature, then dialyzed against 0.1 M sodium acetate buffer, pH 4.6. See Gaertner, H.F. and Offord, R.E. (1996) *supra.*
(3) The N-terminal threonine in G-CSF (at 5 mg/ml), after the aminopeptidase-catalyzed removal of the N-terminal methionine, was oxidized with a 5-fold excess of periodate in 1 % NH₄-HCO₃ buffer, pH 8.3, containing 6 M guanidium hydrochloride for 10 minutes at room temperature. The reaction was stopped by the addition of a 1000-fold molar excess of ethylene glycol over periodate. See Gaertner, H.F. and Offord, R.E. (1996) *supra.*

Preferred methods to oxidize the IFN-β-1b polypeptides include the experimental protocols (1), (2), and (3) above, which result in the oxidation of the N-terminal serine residue of IFN-β-1b, but without any deleterious effect on the biological activity of the polypeptide, as described herein below.

### Analogs, Fragments, Derivatives, and Variants of Improved IFN-β-1b Polypeptides

An analog, fragment, derivative, or variant of the improved IFN-β-1b polypeptides may be: (i) one in which one or more of the amino acid residues are substituted with a conserved or non-conserved amino acid residue (preferably a conserved amino acid residue) and such substituted amino acid residue may or may not be one encoded by the genetic code; or (ii) one in which one or more of the amino acid residues includes a substituent group; or (iii) one in which the improved IFN-β-1b polypeptide is fused with another compound, such as a compound to increase the half-life of the improved IFN-β-1 b polypeptide (for example, polyethylene glycol); or (iv) one in which additional amino acids are fused to the mature, improved IFN-β-1b polypeptide, such as a leader or secretory sequence or a sequence which is employed for purification of the mature, improved IFN-β-1b polypeptide; or (v) one in which the improved IFN-β-1b polypeptide sequence is fused with a larger polypeptide, *i.e*., human albumin, an antibody or Fc, for increased duration of effect. Such analogs, fragments, derivatives, and variants are deemed to be within the scope of those skilled in the art from the teachings herein.

Preferably, the derivatives will contain conservative amino acid substitutions made at one or more predicted, preferably nonessential amino acid residues. A "nonessential" amino acid residue is a residue that can be altered from the wild-type sequence of a protein without altering the biological activity, whereas an "essential" amino acid residue is required for biological activity. A "conservative amino acid substitution" is one in which the amino acid residue is replaced with an amino acid residue having a similar side chain. Families of amino acid residues having similar side chains have been defined in the art. These families include amino acids with basic side chains (*e.g.*, lysine, arginine, histidine), acidic side chains (*e.g.*, aspartic acid, glutamic acid), uncharged polar side chains (*e.g*., glycine, asparagine, glutamine, serine, threonine, tyrosine, cysteine), nonpolar side chains (*e.g*., alanine, valine, leucine, isoleucine, proline, phenylalanine, methionine, tryptophan), beta-branched side chains (*e.g*., threonine, valine, isoleucine) and aromatic side chains (*e.g*., tyrosine, phenylalanine, tryptophan, histidine). Non-conservative substitutions would not be made for conserved amino acid residues or for amino acid residues residing within a conserved protein domain. Fragments or biologically active portions include polypeptide fragments suitable for use as a medicament, as a research reagent, and the like. Fragments include peptides comprising amino acid sequences sufficiently similar to or derived from the amino acid sequences of an improved IFN-β-1b polypeptide and exhibiting at least one activity of that polypeptide, but which include fewer amino acids than the full-length polypeptides disclosed herein. Typically, biologically active portions comprise a domain or motif with at least one activity of the polypeptide. A biologically active portion of a polypeptide can be a peptide that is, for example, 5 or more amino acids in length. Such biologically active portions can be prepared synthetically or by recombinant techniques and can be evaluated for one or more of the functional activities of a polypeptide by means disclosed herein and/or well known in the art.

Preferred derivatives include improved IFN-β-1b polypeptides that have been fused with another compound, such as a compound to increase the half-life of the polypeptide and/or to reduce potential immunogenicity of the polypeptide (for example, polyethylene glycol, "PEG"). The PEG can be used to impart water solubility, size, slow rate of kidney clearance, and reduced immunogenicity to the fusion protein. See, *e.g.,* U.S. Patent No. 6,214,966. In the case of PEGylations, the fusion of the improved IFN-β-1b polypeptide to PEG can be accomplished by any means known to one skilled in the art. For example, PEGylation can be accomplished by first introducing a cysteine mutation into the improved IFN-β-1b polypeptide, followed by site-specific derivatization with PEG-maleimide. The cysteine residue can be added to the C-terminus of the improved IFN-β-1b polypeptide. See, *e.g*., Tsutsumi, Y. et al. (2000) Proc. Natl. Acad. Sci. USA 97:8548-8553.

Variants of the improved IFN-β-1b polypeptides include polypeptides having an amino acid sequence sufficiently similar to the amino acid sequence of the original improved IFN-β-1b polypeptides. The term "sufficiently similar" means a first amino acid sequence that contains a sufficient or minimum number of identical or equivalent amino acid residues relative to a second amino acid sequence such that the first and second amino acid sequences have a common structural domain and/or common functional activity. For example, amino acid sequences that contain a common structural domain that is at least about 45%, preferably about 75% through 98%, identical are defined herein as sufficiently similar. Preferably, variants will be sufficiently similar to the amino acid sequence of the preferred improved IFN-β-1b polypeptides. Variants include variants of improved IFN-β-1b polypeptides encoded by a polynucleotide that hybridizes to a polynucleotide or a complement thereof under stringent conditions. Such variants generally retain the functional activity of the improved IFN-β-1b polypeptides . Libraries of fragments of the polynucleotides can be used to generate a variegated population of fragments for screening and subsequent selection. For example, a library of fragments can be generated by treating a double-stranded PCR fragment of a polynucleotide with a nuclease under conditions wherein nicking occurs only about once per molecule, denaturing the double-stranded DNA, renaturing the DNA to form double-stranded DNA which can include sense/antisense pairs from different nicked products, removing single-stranded portions from reformed duplexes by treatment with S1 nuclease, and ligating the resulting fragment library into an expression vector. By this method, one can derive an expression library that encodes N-terminal and internal fragments of various sizes of the improved IFN-β-1b polypeptides.

Variants include improved IFN-β-1b polypeptides that differ in amino acid sequence due to mutagenesis. Variants with antiviral activity can be identified by screening combinatorial libraries of mutants, for example truncation or point mutants, of improved IFN-β-1b polypeptides using the MxA gene induction assay described under Assays to Measure the Specific Activity of IFN-β-1b Polypeptides.

In one embodiment, a variegated library of variants is generated by combinatorial mutagenesis at the nucleic acid level and is encoded by a variegated gene library. A variegated library of variants can be produced by, for example, enzymatically ligating a mixture of synthetic oligonucleotides into gene sequences such that a degenerate set of potential variant amino acid sequences is expressible as individual polypeptides, or, alternately, as a set of larger improved IFN-β-1b polypeptides (for example, for phage display) containing the set of sequences therein. There are a variety of methods that can be used to produce libraries of potential variants from a degenerate oligonucleotide sequence. Chemical synthesis of a degenerate gene sequence can be performed in an automatic DNA synthesizer, and the synthetic gene then ligated into an appropriate expression vector. Use of a degenerate set of genes allows for the provision, in one mixture, of all of the sequences encoding the desired set of potential variant sequences. Methods for synthesizing degenerate oligonucleotides are known in the art (see, *e.g*., Narang, S.A. (1983) Tetrahedron 39:3; Itakura, K. et al. (1984a) Annu. Rev. Biochem. 53:323-356; Itakura, K. et al. (1984b) Science 198:1056-1063; Ike, Y. et al. (1983) Nucl. Acid Res. 11:477-488).

Several techniques are known in the art for screening gene products of combinatorial libraries made by point mutations or truncation, and for screening cDNA libraries for gene products having a selected property. Such techniques are adaptable for rapid screening of the gene libraries generated by the combinatorial mutagenesis of improved IFN-β-1b polypeptides for antiviral activity. The most widely used techniques, which are amenable to high throughput analysis for screening large gene libraries typically include cloning the gene library into replicable expression vectors, transforming appropriate cells with the resulting library of vectors and expressing the combinatorial genes under conditions in which detection of a desired activity facilitates isolation of the vector encoding the gene whose product was detected. Recursive ensemble mutagenesis (REM), a technique that enhances the frequency of functional mutants in the libraries, can be used in combination with the screening assays to identify the desired variants.

### Assays to Measure the Specific Activity of IFN-β-1b Polypeptides

The antiviral activity of IFNs can be measured quantitatively in terms of reduction in virus yield or virus-induced cytopathic effects ("CPE"), *e.g*., by vital dye uptake, in cultured human cells, as described, for example, in Pestka, S., ed., Interferons Part A in Meth. Enzymol. Vol. 78, Academic Press, Inc., New York, N.Y. (1981).

### Antiviral Cytopathic Effect (CPE) Assay

The specific activity of IFN-β polypeptides, including both IFN-β-1a and IFN-β-1b, can be determined using methods described herein or known in the art, e.g., an antiviral CPE assay. One antiviral CPE assay used herein measures naphthol blue-black dye uptake in A549 human lung carcinoma cells infected by murine encephalomyocarditis ("EMC") virus. This procedure is described in detail in Pungor, E. Jr. et al. (199) J. Interferon Res. 18:1025-1030. Another antiviral CPE assay used herein measures viral plaque reduction in WISH human amnionic cells infected by vesicular stomatitis virus ("VSV"). International Units ("IU") are assigned in terms of a reference NIH human fibroblast IFN-β composition or reference human recombinant IFN-β composition (Pestka, S. (1986) *supra).*

### MxA Gene Induction Assay

Based on the specific induction of the MxA gene by type I IFNs in cultured human cells (Von Wussow, P. et al. (1990) Eur. J. Immunol. 20:2015-2019; and Simon, A. et al. (1991) J. Interferon Res. 11:215-219), an immunochemiluminometric ("ICLA") assay was developed by Files, J.G. et al. (1998) J. Interferon Res. 18:1019-1024. The ICLA assay, which is based on the procedure of Oh, S.K. et al. (1994) J. Immunol. Meth. 176:79-91, detects MXa protein in cell lysates using two non-competing anti-MxA monoclonal antibodies, one of which is conjugated with acridinium ester, which is detected in a luminometer. The MxA gene induction ICLA assay procedure used herein is described in detail in Files, J.G. *et al.* (1998) *supra.*

### Pharmaceutical Compositions

Also described herein are pharmaceutical compositions that can be administered to a patient to achieve a therapeutic effect. Pharmaceutical compositions can be prepared for administration by combining an improved IFN-β-1b polypeptide, having the desired degree of purity and the pharmaceutically effective amount, with physiologically acceptable carriers.

The improved IFN-β-1b polypeptides can be used in pharmaceutical compositions, for intravenous, subcutaneous, intramuscular, or intrathecal administration. Thus, the above described polypeptides preferably will be combined with an acceptable sterile pharmaceutical carrier, such as five percent dextrose, lactated Ringer's solution, normal saline, sterile water, or any other commercially prepared physiological buffer solution designed for intravenous infusion. It will be understood that the selection of the carrier solution, and the dosage and administration of the composition, will vary with the subject and the particular clinical setting, and will be governed by standard medical procedures.

These pharmaceutical compositions may be administered in amounts effective to inhibit or ameliorate the pathological consequences or symptoms associated with MS.

Administration of the improved IFN-β-1b polypeptides may be by bolus intravenous injection, by constant intravenous infusion, or by a combination of both routes. Alternatively, or in addition, the improved IFN-β-1b polypeptides mixed with appropriate excipients may be taken into the circulation via an intramuscular site.

The improved IFN-β-1b polypeptides and pharmaceutical compositions are useful for parenteral, topical, intravenous, oral, or local administration. The pharmaceutical compositions can be administered in a variety of unit dosage forms depending upon the method of administration. For example, unit dosage forms can be administered in the form including, but not limited to, tablets, capsules, powder, solutions, and emulsions.

The improved IFN-β-1b polypeptides and pharmaceutical compositions are particularly useful for intravenous administration. The compositions for administration will commonly comprise a solution of the improved IFN-β-1b polypeptide dissolved in a pharmaceutically acceptable carrier, preferably in an aqueous carrier. A variety of aqueous carriers can be used, e.g., buffered saline and the like. These solutions are sterile and generally free of undesirable matter. The compositions may be sterilized by conventional, well-known sterilization techniques.

A typical pharmaceutical composition for intravenous administration can be readily determined by one of ordinary skill in the art. The amounts administered are clearly protein specific and depend on its potency and pharmacokinetic profile. Actual methods for preparing parenterally administrable compositions will be known or apparent to those skilled in the art and are described in more detail in such publications as Remington's Pharmaceutical Science, 18th ed., Mack Publishing Company, Easton, PA, 1990).

The compositions containing the present improved IFN-β-1b polypeptides or a cocktail thereof (*i.e*., with other proteins) can be administered in therapeutic treatments. In therapeutic applications, compositions are administered to a patient suffering from MS in an amount sufficient to cure or at least partially arrest the disorder. An amount adequate to accomplish this is defined as a "therapeutically effective dose". Amounts effective for this use will depend upon the severity of the disease and the general state of the patient's health.

Single or multiple administration of the compositions may be administered depending on the dosage and frequency as required and tolerated by the patient. In any event, the composition should provide a sufficient quantity of the improved IFN-β-1b polypeptides to effectively treat the patient. Generally, depending on the intended mode of administration, the pharmaceutically acceptable compositions will contain about 1% to about 99% by weight of an improved IFN-β-1b polypeptide , and 99% to 1% by weight of a suitable pharmaceutical excipient or carrier. Preferably, the composition will be about 5% to 75% by weight of an improved IFN-β-1b polypeptide(s) , with the rest being suitable pharmaceutical excipients or carriers.

The improved IFN-β-1b polypeptides , or their pharmaceutically acceptable compositions, are administered in a therapeutically effective amount, which will vary depending upon a variety of factors including the specific activity of the particular improved IFN-β-1b polypeptide employed; the metabolic stability and length of action of the improved IFN-β-1b polypeptide; the age, body weight, general health, sex, and diet of the patient; the mode and time of administration; the rate of excretion; the drug combination; the severity of the particular disease-states; and the host undergoing therapy. Generally, a therapeutically effective daily dose is from about 0.14 mg to about 14.3 mg/kg of body weight per day of an improved IFN-β-1b polypeptide , preferably, from about 0.7 mg to about 10 mg/kg of body weight per day; and most preferably, from about 1.4 mg to about 7.2 mg/kg of body weight per day. For example, for administration to a 70 kg person, the dosage range would be from about 10 mg to about 1.0 gram per day of an improved IFN-β-1b polypeptide , preferably from about 50 mg to about 700 mg per day, and most preferably from about 100 mg to about 500 mg per day.

### Therapeutic Indications

The two forms of IFN-β, IFN-β-1a and IFN-β-1b, are approved for the treatment of relapsing remitting MS (Vartanian, T. (2003) *supra).* IFN-β is also used to treat genital warts. The improved IFN-β-1b polypeptides are useful in the above diseases, disorders, or conditions, and are also useful for the treatment of other forms of MS, including secondary progressive MS, primary progressive MS, and progressive relapsing MS. By useful it is meant that the improved IFN-β-1b polypeptides are useful for treatment of disease, e.g., either to prevent disease, or to prevent disease progression to a more severe state, or to amerliorate or reduce a symptom or consequence of disease, such as MS.

### Preferred Embodiments

Of the improved IFN-β-1b polypeptides as set forth above in the Summary of the Invention, several groups of improved IFN-β-1b polypeptides are particularly preferred.

In one embodiment, the improved IFN-β-1b polypeptides have at least 2-fold greater specific activity than that of a reference IFN-β composition.

In a preferred embodiment, the improved IFN-β-1b polypeptides have at least 4-fold greater specific activity than that of a reference IFN-β composition.

In a more preferred embodiment, the improved IFN-β-1b polypeptides have at least 6-fold greater specific activity than that of a reference IFN-β composition.

In a more preferred embodiment, the improved IFN-β-1b polypeptides have at least 8-fold greater specific activity than that of a reference IFN-β composition.

In an even more preferred embodiment, the improved IFN-β-1b polypeptides have at least 10-fold greater specific activity than that of a reference IFN-β composition.

The following specific examples are provided as a guide to assist in the practice of the invention, and are not intended as a limitation on the scope of the invention.

### EXAMPLE 1

### Comparison of IFN-β-1a and IFN-β-1b Activity In Vitro

Recombinant human IFN-β-1a is a 166 amino acids in length, N-linked glycosylated polypeptide expressed in CHO cells ("IFN-β-1a"). By contrast, recombinant human IFN-β-1b is a 165 amino acids in length, non-glycosylated polypeptide expressed in *E. coli* ("IFN-β-1b"). The N-terminal methionine residue in IFN-β-1a is deleted in IFN-β-1b, so that the N-terminal amino acid in IFN-β-1b is Ser2. In addition, IFN-β-1b has Cys17 replaced by Ser17. Figure 1 shows the amino acid sequences of (A) IFN-β-1a and (B) IFN-β-1b. The specific activity of IFN-β-1a is 2 to 3 x 10⁸ IU/mg, whereas the specific activity of IFN-β-1b is 2 to 3 x 10⁷ IU/mg. The relative contribution of the loss of the N-terminal Met, replacement of Cys17 with Ser17, absence of N-linked glycosylation on Asn80, and exposure to SDS during purification to the observed difference in specific activity between IFN-β-1a and IFN-β-1b was investigated.

IFN-β-1a (obtained from Dr. Rentschler Biotechnologie GmbH) was purified by affinity chromatography using the immunoaffinity chromatography column and procedures described in EP 0529300. IFN-β-1a_{SER17}, in which Cys17 is replaced by Ser17, was constructed using standard recombinant DNA technology (see Mark, D.F. *et al.* (1984) *supra* and U.S. Patent No. 4,588,585), expressed in CHO cells, and then purified by affinity chromatography as described for IFN-β-1a. N-terminal amino acid sequence analysis was performed on an HP G1005A protein sequencer following the standard recommended protocol. The N-terminal amino acid residue of IFN-β-1a was Met 1, as expected, whereas the N-terminal amino acid residue of >80% of the IFN-β-1a_{SER17} molecules was Ser2, similar to IFN-β-1b. The effect of the combined loss of the N-terminal Met1 and replacement of Cys17 with Ser17 was assessed in the MxA gene induction assay, as described under Assays to Measure the Specific Activity of IFN-β-1b Polypeptides. As shown in Figure 2A, using the product label specific activity of reconstituted IFN-β-1b/Betaseron^{®} as IFN standard, the specific activity of IFN-β-1a was 1.45 to 3.22 x 10⁸ IU/mg, whereas the specific activity of IFN-β-1a_{SER17} was 1.3 to 2.4 x 10⁸ IU/mg.

IFN-β-1a_{SER17} was deglycosylated in non-reducing (1% SDS; 0.5% NP-40; and 100 mM Tris-HCl, pH 7.6) and reducing (1% SDS; 0.5% NP-40; 100 mM Tris-HCl, pH 7.6; 140 mM β-mercaptoethanol; and -10 minutes boiling, followed by cooling to room temperature) conditions. Non-reduced and reduced IFN-β-1a_{SER17} were incubated overnight (18 hours) with peptide N-glycosidase F ("PNGase F"). The reduced, deglycosylated IFN-β-1a_{SER17} was allowed to spontaneously reoxidize in air for up to 18 hours. The complete deglycosylation of IFN-β-1a_{SER17} was verified by Western blot analysis, using IFN-β-1b, with and without reduction and alkylation, as migration standards. As shown in Figure 2B, using the MxA gene induction assay, the specific activity of IFN-β-1a_{SER17} was not adversely affected by overnight incubation alone. Reduction of IFN-β-1a_{SER17} in the presence of the strong denaturant SDS, followed by its spontaneous reoxidation, led to ∼20% reduction in specific activity. The PNGase F-mediated deglycosylation of IFN-β-1a_{SER17}, under either non-reducing or reducing conditions, led to a 30-35% decrease in specific activity.

In summary, neither the combined loss of N-terminal Met1 and replacement of Cys17 with Ser17, nor deglycosylation at Asn80 in the absence or presence of the strong denaturant SDS, accounts for the -10-fold greater specific activity of IFN-β-1a as compared to IFN-β-1b.

### EXAMPLE 2

### Ganglioside-Mediated Inhibition of IFN-β-1b Activity In Vitro

Experiments using partially purified fibroblast IFN-β preparations indicated that gangliosides inhibit the antiviral activity of native IFN-β *in vitro* (Gupta, S.L. *et al.* (1984) *supra;* and Fuse, A. *et al.* (1982) *supra*). The effect of gangliosides on the biological activity of IFN-β-1b *in vitro* was investigated. IFN-β-1b was prepared as described in U.S. Patent No. 5,814,485.

A variety of gangliosides are present in human serum or plasma. See, for example, Kundu, S.K. et al. (1985) Arch. Biochem. Biophys. 238:388-400; and Senn, H.J. *et al.* (1989) *supra.* Figure 3 shows the pathways involved in the generation of gangliosides starting from GM₃ (N-acetylneuraminosyl)galactosylglucosylceramide).

IFN-β-1b, in the presence of increasing amounts of gangliosides GM₃, GM₂, GM₁, GD₁ₐ, GD₃, GD_{1b}, and GT_{1b}, was tested in the MxA gene induction assay, as described under Assays to Measure the Specific Activity of IFN-β-1b Polypeptides. As shown in Figure 3, several of these gangliosides, in the low micromolar (µM) range, were able to inhibit the ability of IFN-β-1b to induce the MxA gene by 50%. In general, the gangliosides with more potent inhibitory activity were those with more complex structures.

### EXAMPLE 3

### Presence of Carbohydrate in IFN-β-1b Preparations

Recombinant human IFN-β-1b/Betaseron^{®}, as described in U.S. Patent No. 4,588,585, is produced in *E*. *coli* according to the procedure described in U.S. Patent No. 5,814,485. See Lin, L. (1998) *supra* for review. The final step in the IFN-β-1b purification prior to formulation is column chromatography on Sephedex G-75, a bead-formed, crosslinked dextran gel filtration medium. The G-75 pool material was used herein to investigate the presence of carbohydrate in preparations of IFN-β-1b purified as described in U.S. Patent No. 5,814,485. Two lots of IFN-β-1b were tested for their monosaccharide content.

### Monosaccharide Compositions of Oligosaccharides in the IFN-β-1b G-75 Pool Material

IFN-β-1b was hydrolyzed in either 6 M HCl or 2 M TFA at 100°C for 1 hour, dried in a speed vac, and reconstituted in water. Monosaccharide composition analysis was performed by high performance anion exchange chromatography combined with pulse electrochemical detection on a Dionex Glycostation (Dionex, Inc.), using a Carbopack PA-1 anion exchange column and a Carbopack guard column. The Glycostation was calibrated with a mixture of fucose, *N*-acetylgalactosamine, *N*-acetylglucosamine, galactose, glucose, and mannose. The monosaccharides were eluted in an isocratic mode (16 mM NaOH), and the column was washed with 200 mM NaOH to elute oligosaccharides and other adsorbed anionic species.

Using 6 M HCl acid hydrolysis, all six monosaccharides were present in IFN-β-1b lot A, and fucose, galactose, and mannose were present in IFN-β-1b lot B (Figure 4A). The molar ratios of total monosaccharides to IFN-β-1b were 0.58 and 0.05 in lots A and B, respectively. These ratios are considered to be lower estimates of carbohydrate content in the IFN-β-1b lots since the acid hydrolysis is not complete and some monosaccharides degrade during the acid hydrolysis. See Beeley, J.G. in Laboratory Techniques in Biochemistry and Molecular Biology, Elsevier, New York, N.Y. (1985). Using 2 M TFA acid hydrolysis, fucose, *N*-acetylglucosamine, glucose, and mannose were present in IFN-β-1b lot A, and fucose, *N*-acetylglucosamine, and galactose were present in IFN-β-1b lot B (Figure 4A). The molar ratios of total monosaccharides to IFN-β-1b were 0.09 and 0.08 in lots A and B, respectively. This result is in agreement with published data that acid hydrolysis in TFA is milder than in HCl (Beeley, J.G. (1985) *supra).*

Of the two possible carbohydrate sources, the presence of glucose in IFN-β-1b lots is best explained by resin leaching from the Sephacryl S-200 and Sephadex G-75 dextranbased columns used in the purification procedure. In contrast, the non-glucose residues most likely arise from the host *E*. *coli.*

### Protein-Oligosaccharide Interactions and Separations

The nature of the IFN-β-1b protein-carbohydrate interaction was explored by column chromatography. IFN-β-1b lot A was further purified by reverse phase high pressure liquid chromatography (RP-HPLC) using Vydac C4 columns in a water, acetonitrile, and TFA solvent system. After chromatography, the IFN-β-1b was hydrolyzed in 6 M HCl and monosaccharide analysis was performed as described above. Figure 4B shows that RP-HPLC did not remove the carbohydrates from IFN-β-1b lot A. The distribution of the identified monosaccharides changed, however, possibly due to the different solvents employed: 50 mM sodium acetate in 0.1% SDS in the G-75 pool, as compared to acetonitrile/TFA in the RP-HPLC.

Alternatively, IFN-β-1b lot A was further purified over NAP 10 and Sephadex G-25 size exclusion columns, with 5 mM HCl and 1.5 mM NaOH in the mobile phases, respectively. Figure 4B shows that neither NAP 10 nor G-25 size exclusion chromatography removed carbohydrates from IFN-β-1b lot A, although, as was found for RP-HPLC, the distribution of the identified monosaccharides changed. The quantitative information should be handled with caution, since the solvents are different for each chromatographic system and the extent of oligosaccharide hydrolysis differed in each case.

IFN-β-1b lot A was also further purified by Concanavalin A ("Con A") Sepharose column chromatography using the Pharmacia FPLC solvent delivery and UV detector systems. The Con A Sepharose column was equilibrated in Buffer A (20 mM Tris-HCl, pH 7.4; 500 mM NaCl, 1 mM CaCl₂, and 1 mM MnCl₂), loaded with 5 mg IFN-β-1b lot A, and then re-equilibrated and extensively washed with Buffer A. Elution was performed by continuous gradient between Buffer A and Buffer B (Buffer A + 500 mM α-methyl-D-mannose pyranoside). Samples of gradient fractions were dialyzed against water, dried in a speed vac, and either reconstituted in 50 mM sodium acetate in 0.1% SDS or reconstituted in 6 M HCl, and subjected to monosaccharide analysis as described above.

Figure 4B shows that the peak IFN-β-1b (fraction 5) from the Con A Sepharose column contains glucose, which leaches from the column, and mannose. The Con A Sepharose column effectively removed the amino sugars from the IFN-β-1b lot A material. The majority of oligosaccharides present in the IFN-β-1b starting material was removed by Con A Sepharose column chromatography, indicating that the IFN-β-1b protein-carbohydrate interaction is predominantly, if not completely, non-covalent.

The peak IFN-β-1b (fraction 5) from the Con A Sepharose column was next tested for antiviral activity. Using the WISH CPE assay, which measures viral plaque reduction by type I IFNs in the human amnionic cell line WISH infected with VSV, the specific activity was 3.6 x 10⁷ IU/mg. This specific activity is at least similar to that of the IFN-β-1b starting material, indicating that the removal of carbohydrates from IFN-β-1b preparations may be beneficial.

### Characterization of Oligosaccharides by Lectin Binding

Finally, IFN-β-1b lot A was tested for binding to biotinylated lectins from Vector Laboratories. Of the six lectins tested, Con A, Datura Stramonium Lectin ("DSL"), Jacalin, Soybean Agglutinun ("SBA"), Maackia Amurensis Lectin II ("MAL II"), and Dolichos Bifluorus Agglutinin ("DNA"), only DSL did not react with IFN-β-1b that was dot blotted onto nitrocellulose. This result is consistent with the above monosaccharide composition analysis. The peak IFN-β-1b (fraction 5) also no longer reacted with SBA, consistent with the effective removal of carbohydrates from the IFN-β-1b lot A material.

In summary, IFN-β-1b preparations contain readily detectable carbohydrate in the form of oligosaccharides, which are predominantly, if not completely, non-covalently attached to purified IFN-β-1b. The quantity and quality of the carbohydrate appears to vary between IFN-β-1b preparations. The source of carbohydrate is the host *E*. *coli* production strain and not the purification procedure itself. The carbohydrate can be largely removed by Con A Sepharose column chromatography, and its removal does not adversely affect, and may possibly be beneficial for, the biological activity of IFN-β-1b.

### EXAMPLE 4

### Biological Activity of Oxidized IFN-β-1b In Vitro

IFN-β-1b, expressed and purified as described in U.S. Patent No. 5,814,485, is treated with the oxidizing agent sodium periodate under a variety of experimental conditions, as described under Methods of Oxidation of Improved IFN-β-1b Polypeptides. The specific activity of oxidized FN-β-1b is determined using the MxA gene induction assay *in vitro,* as described under Assays to Measure the Specific Activity of IFN-β-1b Polypeptides. Three examples of recombinant mammalian cytokines that have been oxidized with sodium periodate, without adversely affecting their biological activity, include IL-8, G-CSF, and IL-1α (Geoghegan, K.F. and Stroh, J.G. (1992) *supra;* and Gaertner, H.F. and Offord, R.E. (1996) *supra*).

After oxidation, IFN-β-1b is repurified by column chromatography, for example, on a Sephadex G-75 size exclusion column, as described in U.S. Patent No. 5,814,485. Removal of carbohydrate from the oxidized IFN-β-1b preparations is evaluated by the analysis of their monosaccharide composition after acid hydrolysis in 6 M HCl, as described in Example 3. The specific activity of the oxidized IFN-β-1b preparations is then determined using the MxA gene induction assay.

In this *in vitro* assay, the oxidized IFN-β-1b qualitatively displays the same biological function as IFN-β-1b. When oxidized in this manner, the specific activity of oxidized IFN-β-1 b is increased at least 2-fold, preferably at least 4-fold, more preferably at least 6-fold, and even more preferably 10-fold, as compared to IFN-β-1b.

### EXAMPLE 5

### Biological Activity of Oxidized IFN-β-1b In Vivo

Experimental allergic encephalomyelitis ("EAE") in rodents is widely used as a model for MS. See, for example, Swanborg, G. (1995) Clin. Immunol. Immunopathol. 77:4-13; and Martin, R. and McFarland, H. (1996) Springer Semin. Immunopathol. 18:1-24. IFN-β-1b shows *in vivo* efficacy in these relevant MS models. Using these models, the oxidized IFN-β-1b of this invention demonstrates qualitatively the same biological function as IFN-β-1b that is expressed and purified as described in U.S. Patent No. 5,814,485.

### Passive Transfer Experimental Allergic Encephalomyelitis in SJL Mice

Animals and materials: 8 week old female SJL mice (Jackson Laboratories); RPMI 1640, with L-glutamine and 25 mM HEPES, 1X, 0.1 micron filtered (Life Technologies); FBS, defined (Hyclone, heat inactivated); MEM non-essential amino acids solution, 10 mM, 100X (Life Technologies); 2-β-mercaptoethanol, 1000X, 5.5 x 10⁻² M in D-PBS (Life Technologies); Penicillin/Streptomycin ("Pen/Strep"), 10,000 U/µg per ml (Bio-Whittaker); Hank's Balanced Salt Solution, 1X, 0.1 micron filtered (Life Technologies).

Eight-week-old female SJL mice are immunized with 0.1 ml subcutaneous (divided between base of tail and upper back) injection containing 150 µg proteolipid protein ("PLP") in complete Freund's adjuvant ("CFA") with 200 µg *Mycobacterium tuberculosis* H37Ra (ground). Eleven days later, axial, brachial and inguinal lymph node cells are excised from the mice and cultured at 6X10⁶ cells/ml in the following media (to 450 ml RPMI 1640 (with L-glutamine plus HEPES) add 50 ml FBS, 0.455 ml 2-β-mercaptoethanol, 5.0 ml Pen/Strep and 5.0 ml non-essential amino acids. PLP is added to the cells to obtain a final concentration of 50 µg/ml. Cells are incubated for 72 hours at 37°C, 7% CO₂. Cells are harvested and washed twice in HBSS. Viability of the lymph node cells is assessed by Trypan Blue exclusion. The concentration of lymph node cells is adjusted to 4 x 10⁷ cells per ml. 2 x 10⁷ lymph node cells are injected intraperitoneally (dose volume = 0.5 ml) per mouse into naive 8 week old female SJL mice. The mice are weighed and scored daily. Treatment with IFN-β-1b and oxidized IFN-β-1b is administered as needed. Clinical evaluation (EAE score/symptoms): 0/normal; 1/limp tail; 2/difficulty righting; 3/incomplete paralysis of one or both hind limbs; 4/complete paralysis of one or both hind limbs; 5/immobile, moribund or dead.

### Acute Experimental Allergic Encephalomyelitis in Lewis Rats

Animals and materials: female Lewis rats (Charles River), immunized at 8 weeks of age; spinal cord homogenate preparation (from male Hartley guinea pigs, Simonsen Labs): 500-700 g guinea pigs are euthanized with CO₂. The spinal cords are removed using sharp bone scissors to cut the vertebrae, rinsed in saline, blotted once, and stored at -80°C until the day of use. Spinal cords are then weighed and homogenized with saline at 1 g/ml of saline; antigen emulsion: guinea pig spinal cord homogenate is mixed 1:1 with CFA (Difco) with 1 mg/ml *Mycobacterium tuberculosis* (ground with a mortar and pestle). 0.05 ml is injected into each hind limb footpad for a total of 0.1 ml per rat.

Eight-week old female Lewis rats are immunized with a single bolus injection on day 1. Rats are weighed and scored daily. Treatment with IFN-β-1b and oxidized IFN-β-1b is administered as needed. Clinical Evaluation (EAE score/symptoms): 0/normal; 1/limp tail; 2/incomplete paralysis of one or both hind limbs; 3/ complete paralysis of one hind limb or both hind limbs can move but do not help in movement of the body; 4/complete paralysis of both hind limbs; 5/complete paralysis of hind limbs and weakness of one or both forelimbs or moribund, or death.

In these *in vivo* assays, the oxidized IFN-β-1b displays qualitatively the same biological function as IFN-β-1b. When oxidized in this manner, the specific activity of oxidized IFN-β-1b is increased at least 2-fold, preferably at least 4-fold, more preferably at least 6-fold, and even more preferably at least 10-fold, as compared to IFN-β-1b.

### EXAMPLE 6

This example illustrates the preparation of representative pharmaceutical compositions for oral administration containing an improved IFN-β-1b polypeptide of the invention, or a pharmaceutically acceptable salt thereof:

| A. | Ingredients | % wt./wt. |
|---|---|---|
| | Improved IFN-β-1b polypeptide of the invention | 20.0% |
| | Lactose | 79.5% |
| | Magnesium stearate | 0.5% |

The above ingredients are mixed and dispensed into hard-shell gelatin capsules containing 100 mg each, one capsule would approximate a total daily dosage.

| B. | Ingredients | % wt./wt. |
|---|---|---|
| | Improved IFN-β-1b polypeptide of the invention | 20.0% |
| | Magnesium stearate | 0.9% |
| | Starch | 8.6% |
| | Lactose | 69.6% |
| | PVP (polyvinylpyrrolidine) | 0.9% |

The above ingredients with the exception of the magnesium stearate are combined and granulated using water as a granulating liquid. The formulation is then dried, mixed with the magnesium stearate and formed into tablets with an appropriate tableting machine.

| C. | Ingredients | |
|---|---|---|
| | Improved IFN-β-1b polypeptide of the invention | 0.1 g |
| | Propylene glycol | 20.0 g |
| | Polyethylene glycol 400 | 20.0 g |
| | Polysorbate 80 | 1.0 g |
| | Water | q.s. 100 mL |

The improved IFN-β-1b polypeptide of the invention is dissolved in propylene glycol, polyethylene glycol 400 and polysorbate 80. A sufficient quantity of water is then added with stirring to provide 100 mL of the solution, which is filtered and bottled.

| D. | Ingredients | % wt./wt. |
|---|---|---|
| | Improved IFN-β-1b polypeptide of the invention | 20.0% |
| | Peanut Oil | 78.0% |
| | Span 60 | 2.0% |

The above ingredients are melted, mixed and filled into soft elastic capsules.

| E. | Ingredients | % wt./wt. |
|---|---|---|
| | Improved IFN-β-1b polypeptide of the invention | 1.0% |
| | Methyl or carboxymethyl cellulose | 2.0% |
| | 0.9% saline | q.s. 100 mL |

The improved IFN-β-1b polypeptide of the invention is dissolved in the cellulose/saline solution, filtered and bottled for use.

### EXAMPLE 7

This example illustrates the preparation of a representative pharmaceutical formulation for parenteral administration containing an improved IFN-β-1b polypeptide of the invention, or a pharmaceutically acceptable salt thereof:

| Ingredients | |
|---|---|
| Improved IFN-β-1b polypeptide of the invention | 0.02 g |
| Propylene glycol | 20.0 g |
| Polyethylene glycol 400 | 20.0 g |
| Polysorbate 80 | 1.0 g |
| 0.9% Saline solution | q.s. 100 mL |

The improved IFN-β-1b polypeptide of the invention is dissolved in propylene glycol, polyethylene glycol 400 and polysorbate 80. A sufficient quantity of 0.9% saline solution is then added with stirring to provide 100 mL of the I.V. solution, which is filtered through a 0.2 µm membrane filter and packaged under sterile conditions.

### EXAMPLE 8

This example illustrates the preparation of a representative pharmaceutical composition in suppository form containing an improved IFN-β-1b polypeptide of the invention, or a pharmaceutically acceptable salt thereof:

| Ingredients | % wt./wt. |
|---|---|
| Improved IFN-β-1b polypeptide of the invention | 1.0% |
| Polyethylene glycol 1000 | 74.5% |
| Polyethylene glycol 4000 | 24.5% |

The ingredients are melted together and mixed on a steam bath, and poured into molds containing 2.5 g total weight.

### EXAMPLE 9

This example illustrates the preparation of a representative pharmaceutical formulation for insufflation containing an improved IFN-β-1b polypeptide of the invention, or a pharmaceutically acceptable salt thereof:

| Ingredients | % wt./wt. |
|---|---|
| Micronized improved IFN-β-1b polypeptide of the invention | 1.0% |
| Micronized lactose | 99.0% |

The ingredients are milled, mixed, and packaged in an insufflator equipped with a dosing pump.

### EXAMPLE 10

This example illustrates the preparation of a representative pharmaceutical formulation in nebulized form containing an improved IFN-β-1b polypeptide of the invention, or a pharmaceutically acceptable salt thereof:

| Ingredients | % wt./wt. |
|---|---|
| Improved IFN-β-1b polypeptide of the invention | 0.005% |
| Water | 89.995% |
| Ethanol | 10.000% |

The improved IFN-β-1b polypeptide of the invention is dissolved in ethanol and blended with water. The formulation is then packaged in a nebulizer equipped with a dosing pump.

### EXAMPLE 11

This example illustrates the preparation of a representative pharmaceutical formulation in aerosol form containing an improved IFN-β-1b polypeptide of the invention, or a pharmaceutically acceptable salt thereof:

| Ingredients | % wt./wt. |
|---|---|
| Improved IFN-β**-**1b polypeptide of the invention | 0.10% |
| Propellant 11/12 | 98.90% |
| Oleic acid | 1.00% |

The improved IFN-β-1b polypeptide of the invention is dispersed in oleic acid and the propellants. The resulting mixture is then poured into an aerosol container fitted with a metering valve.

## Claims

1. A process comprising decreasing the ganglioside inhibitory activity in a composition comprising an IFN-β-1b polypeptide to obtain a composition that has a greater IFN-β-1b specific activity as compared to a reference IFN-β composition, wherein said decreasing of said ganglioside inhibitory activity is by selective oxidation of said IFN-β-1b polypeptide,
wherein said process comprises removing gangliosides from said composition such that it is substantially free of gangliosides.

2. The process according to Claims 1 or 2, wherein said selective oxidation is by heat, chemical, or enzymatic oxidation.

3. The process according to Claim 3, wherein said chemical oxidation is by an oxidizing agent selected from a group consisting of sodium periodate, *o*-iodosobenzoic acid, CuCl₂, and *o*-phenanthroline.

## Patentansprüche

1. Verfahren umfassend das Vermindern der Gangliosid-inhibitorischen Aktivität in einer Zusammensetzung umfassend ein IFN-ß-1b-Polypeptid, um eine Zusammensetzung zu erhalten, die eine im Vergleich zu einer IFN-ß-Referenzzusammensetzung höhere IFN-ß-1b-spezifische Aktivität aufweist, wobei das Vermindern der Gangliosid-inhibitorischen Aktivität durch selektive Oxidation des IFN-ß-1b-Polypeptids erfolgt, wobei das Verfahren das Entfernen von Gangliosiden aus der Zusammensetzung umfasst, derart, dass sie im Wesentlichen frei von Gangliosiden ist.

2. Verfahren nach Anspruch 1 oder 2, wobei die selektive Oxidation durch Wärme, chemische oder enzymatische Oxidation erfolgt.

3. Verfahren nach Anspruch 2, wobei die chemische Oxidation durch ein Oxidationsmittel erfolgt, das ausgewählt ist aus der Gruppe bestehend aus Natriumperiodat, o-lodosobenzoesäure, CuCl₂, und o-Phenanthrolin.

## Revendications

1. Procédé comprenant l'étape de diminuer l'activité inhibitrice de ganglioside dans une composition comprenant un polypeptide IFN-ß-1b pour obtenir une composition qui a une activité IFN-ß-1b spécifique plus haute en comparaison à une composition IFN-ß de référence, dans lequel ladite diminution de l'activité inhibitrice de ganglioside est faite par l'oxydation sélective dudit polypeptide IFN-ß-1b,
dans lequel ledit procédé comprend l'étape d'enlever des gangliosides de ladite composition tel qu'elle est sensiblement sans des gangliosides.

2. Procédé selon la revendication 1 ou 2 dans lequel ladite oxydation sélective est faite par la chaleur, l'oxydation chimique ou enzymatique.

3. Procédé selon la revendication 2 dans lequel ladite oxydation chimique est faite par un agent oxydant sélectionné parmi un groupe consistant en le periodate de sodium, l'acide iodosobenzoïque, le CuCl₂ et l'o-phenantroline.
